# EUROPEAN PATENT APPLICATION

(11) **EP 1 284 141 A2**
(43) Date of publication of application: **19.02.2003**
(21) Application number: 02255450.5
(22) Date of filing: 05.08.2002
(51) Int. Cl.: A61K 31/4418, A61K 45/06, A61P 25/00, A61P 25/28

(54) **Pharmaceutical combinations comprising neuronal nitric oxide synthase inhibitors for the treatment of neurodegenerative diseases**

(30) Priority: 15.08.2001 US 312606 P
(71) Applicant: Pfizer Products Inc., Groton, Connecticut 06340 (US)
(72) Inventor: Lowe III, John Adams, c/o Pfizer Global Research, Groton, Connecticut 06340 (US); Volkmann, Robert A., c/o Pfizer Global Research, Groton, Connecticut 06340 (US)
(74) Representative: England, Peter Michael

(57) **Abstract**

This invention relates to methods of treating neurodegenerative diseases, comprising administering to a patient in need of such treatment an N-NOS inhibitor in combination with either: (a) NMDA receptor antagonist; (b) a sodium channel antagonist; (c) an acetylcholine esterase inhibitor; (d) a dopamine agonist; (e) a potassium channel opener; (f) an AMPA/kainate receptor antagonist; (g) a calcium channel antagonist; (h) a GABA-A receptor modulator (*e.g.*, a GABA-A receptor agonist); (i) TPA (j) matrix-metalloprotease inhibitor or (k) L-Dopa. The pharmaceutical compounds are also disclosed.

## Description

### Background of the Invention

This invention relates to methods of treating neurodegenerative diseases, comprising administering to a patient in need of such treatment selective N-NOS inhibitors (nitric oxide synthase inhibitors) in combination with one or more other compounds that protect neurons from toxic insult, inhibit the inflammatory reaction after brain damage or promote cerebral reperfusion.

More specifically, this invention relates to methods of treating neurodegenerative diseases selected from the group consisting of stroke, hypovolemic shock, traumatic shock, reperfusion injury, multiple sclerosis, AIDS, associated dementia; neuron toxicity, Alzheimers disease, head trauma, adult respiratory disease (ARDS), acute spiral cord injury, Huntington's disease, and Parkinson's Disease comprising administering to a patient in need of such treatment an N-Nitric Oxide Synthase inhibitor [N-NOS inhibitor] in combination with either: (a) L-Dopa; (b) a sodium channel antagonist; (c) a selective N-methyl D-aspartate (NMDA) receptor antagonist (d) a dopamine agonist (e) a potassium channel opener; (f) an AMPA/kainate receptor antagonist; (g) a calcium channel antagonist; (h) a GABA-A receptor modulator (e.g., a GABA-A receptor agonist); (i) an acetyl-choline esterase inhibitor; (j) a matrix metalloprotease (MMP) inhibitor or (k) TPA.

There are three known isoforms of NOS - an inducible form (I-NOS) and two constitutive forms referred to as, respectively, neuronal NOS (N-NOS) and endothelial NOS (E-NOS). Each of these enzymes carries out the conversion of arginine to citrulline while producing a molecule of nitric oxide (NO) in response to various stimuli. It is believed that excess nitric oxide (NO) production by NOS plays a role in the pathology of a number of disorders and conditions in mammals. For example, NO produced by I-NOS is thought to play a role in diseases that involve systemic hypotension such as toxic shock and therapy with certain cytokines. It has been shown that cancer patients treated with cytokines such as interleukin 1 (IL-1), interleukin 2 (IL-2) or tumor necrosis factor (TNF) suffer cytokine-induced shock and hypotension due to NO produced from macrophages, i.e., inducible NOS (I-NOS), see Chemical & Engineering News, Dec. 20, p. 33, (1993). I-NOS inhibitors can reverse this. It is also believed that I-NOS plays a role in the pathology of diseases of the central nervous system such as ischemia. For example, inhibition of I-NOS has been shown to ameliorate cerebral ischemic damage in rats, see Am. J. Physiol., **268,** p. R286 (1995)). Suppression of adjuvant induced arthritis by selective inhibition of I-NOS is reported in Eur. J. Pharmacol., **273,** p. 15-24 (1995).

NO produced by N-NOS is thought to play a role in diseases such as cerebral ischemia, pain, and opiate tolerance. For example, inhibition of N-NOS decreases infarct volume after proximal middle cerebral artery occlusion in the rat, see J. Cerebr. Blood Flow Metab., **14**, p. 924-929 (1994). N-NOS inhibition has also been shown to be effective in antinociception, as evidenced by activity in the late phase of the formalin-induced hindpaw licking and acetic acid-induced abdominal constriction assays, see Br. J. Pharmacol., **110,** p. 219-224 (1993). Finally, opioid withdrawal in rodents has been reported to be reduced by N-NOS inhibition, see Neuropsychopharmacol., **13**, p. 269-293 (1995).

Brain and spinal cord injury caused by neurodegenerative diseases often result in lifelong disability and premature death. The cause of disability and death is the disruption of function and frank death of neurons and other cells in the central nervous system. Therefore, a clear benefit is anticipated from therapies that reduce or prevent neuronal dysfunction and death after ischemic, hypoxic or traumatic CNS insult.

One of the causes of neuronal dysfunction and death after CNS insult is toxicity caused by a prolonged elevation of glutamate and other excitatory amino acids (EAAs) and overactivation of the *N*-methyl-D-aspartate (NMDA) subtype of glutamate receptors. Glutamate and other EAAs play dual roles in the central nervous system as essential amino acids and the principal excitatory neurotransmitters. There are at least four classes of EAA receptors, specifically NMDA, AMPA (2-amino-3-(methyl-3-hydroxyisoxazol-4-yl)propanoic acid), kainate and metabotropic. These EAA receptors mediate a wide range of signaling events that impact all physiological brain functions. As neurotransmitters, EAAs are released from postsynaptic nerve terminals and then are rapidly resequestered by a variety of cellular reuptake mechanisms. Consequently, the physiological levels of EAAs in the brain parenchyma are maintained at a low level. However, after a CNS insult, the levels of EAAs in the parenchyma increase dramatically and may remain elevated for periods of hours to days. This results in pathological overactivation of EAA receptors and neuronal dysfunction and death.

Several lines of evidence suggest that the NMDA subtype of glutamate receptor is the principal mediator of the EAA-induced toxicity described above. Neurons in primary culture are exquisitely sensitive to the toxic effects of NMDA receptor activation and NMDA receptor antagonists protect cultured neurons from both NMDA and glutamate toxicity (Choi et *al*., *J. Neurosci.,* 1988, 8, 185-196; Rosenberg et *al*., 1989, *Neurosci. Lett. 103, 162).* NMDA receptors are also implicated as mediators of neurotoxicity *in vivo* since NMDA receptor antagonists can reduce neuron loss in animal models of focal ischemia (McCulloch, *J*. *Neural. Trans.,* 1994, 71-79) and head trauma (Bullock *et al*., *Acta Neurochir.,* 1992, 55, 49-55). The neuroprotective effect of NMDA receptor inhibition is realized with several different classes of compounds that target different sites on the NMDA receptor-channel complex. These include competitive antagonists at the glutamate binding site such as (R,E)-4-(3-phosphonoprop-2-enyl) piperazine-2-carboxylic acid (d-CPPene) (Lowe *et al.,* 1994, *Neurochem Int. 25,* 583) and *cis*-4-phosphonomethyl-2-piperidine carboxylic acid (CGS-19,755) (Murphy *et al*., 1988, *Br. J. Pharmacol. 95,* 932) and competitive antagonists at the glycine co-agonist (Johnson *et al*., *Nature,* 1987, *327,* 529-531; and Kemp *et al*., *Trends Pharmacol. Sci.,* 1993, *14,* 20-25) binding site such as 5,7-dichloro-4S-(3-phenyl-ureido)-1,2,3,4-tetrahydro-quinoline-2R-carboxylic acid (L-689,560) and 5-nitro-6,7-dichloro-1,4-dihydro-2,3-quinoxalinedione (ACEA-1021) (Leeson *et al*., 1994, *J. Med. Chem. 37,* 4053). Compounds have also been identified which block the NMDA receptor-gated ion channel, including phencyclidine (PCP), (+)-5-methyl-10,11-dihydro-5-H-dibenzo[a,d]cycloheptan-5,10-imine (MK-801) (Kemp *et al.,* 1987, *Trends in Neurosci. 10,* 294), and C-(1-napthyl-*N*'-(3-ethyl phenyl)-*N*'-methyl guanidine hydrochloride (CNS-1102) (Reddy *et al.*, 1994, *J. Med. Chem. 37,* 260).

The neuroprotective effect of NMDA receptor antagonists in experimental systems has prompted considerable interest in the therapeutic potential of this type of compound. Several prototype antagonists have been progressed into clinical trials, especially for stroke and head trauma (Muir *et al.*, 1995, *Stroke 26,* 503-513). However, side effects at therapeutic drug levels have been a significant problem that has hindered the development process (Muir *et al., supra*). In particular, both glutamate competitive antagonists and channel blocking agents cause cardiovascular effects and psychotic symptoms in man. Although the physiological basis for these side effects are not yet understood, in rodents these types of compounds also cause locomotor hyperactivity and a paradoxical neuronal hyperexcitability manifest as neuronal vacuolization in cingulate and retrosplenial cortices (Olney *et al*., 1991, *Science, 254*, 1515-1518). Antagonists at the glycine coagonist site cause less locomotor activation and do not cause neuronal vacuolization at neuroprotective doses in rodents, suggesting that this class of antagonists may be better tolerated in man (Kemp *et al.,* 1993, *Trends Pharmacol. Sci. 14,* 20-25). Unfortunately, physicochemical problems associated with the quinoxalinedione nucleus (solubility, brain penetration, protein binding) have hindered efforts to bring this class forward in the clinic.

The present invention relates to the additional therapeutic benefits that may be gained by treating neurodegenerative disease with an N-NOS inhibitor in combination with other types of compounds. These include compounds that protect neurons from toxic insult, inhibit the inflammatory reaction after brain damage and/or promote cerebral reperfusion. By reducing the pathological consequences of these additional mechanisms, the overall benefit of the therapeutic intervention may be increased. Furthermore, inhibiting multiple pathological processes may provide an unexpected synergistic benefit over and above that which may be achievable alone with the use of an N-NOS inhibitor.

During the course of a neurodegenerative disease a number of toxic products are formed which can further damage brain cells injured by the primary pathological process or produce damage in cells that otherwise escape damage from the primary insult. These toxins include, but are not limited to: nitric oxide (NO); other reactive oxygen and nitrogen intermediates such as superoxide and peroxynitrite; lipid peroxides; TNFα, IL-1 and other interleukins, cytokines or chemokines; cyclooygenase and lipoxygenase derivatives and other fatty acid mediators such as leukotrienes, glutamate and prostaglandins; and hydrogen ions. Inhibiting the formation, action or accelerating the removal of these toxins may protect CNS cells from damage during neurodegenerative disease. Furthermore, the beneficial effects of inhibiting the formation, action or accelerating the removal of these toxins may be additive or synergistic with the benefits of inhibiting nitric oxide synthase. Examples of compounds that inhibit the formation or action of these toxins, or accelerate their removal include, but are not limited to, L-Dopa, a dopamine agonist, sodium channel antagonists, an acetylcholinesterase inhibitor, potassium channel openers, TPA, a matrix metalloprotease inhibitor, an AMPA/kainate receptor antagonists, calcium channel antagonists, GABA-A receptor modulators (*e.g.,* GABA-A receptor agonists), and selective NMDA receptor antagonists.

The formation and release of many of the toxins listed above are triggered by physiological signaling mechanisms that become pathologically activated by neurodegenerative diseases. Activation of these signaling mechanisms can also result in cellular depolarization. This depolarization may disrupt cellular ionic homeostasis, accelerate the rate of energy utilization as the cell strives to maintain homeostasis, and/or further accelerate the rate of formation and release of toxins. Thus, inhibition of these signaling mechanisms during neurodegenerative disease may reduce the degree of cellular dysfunction and death. Furthermore, the beneficial effects of inhibiting these signaling mechanisms may be additive or synergistic with benefits of inhibiting nitric oxide synthase. These signaling mechanisms include, but are not limited to: NMDA receptors, other EAA receptors such as AMPA, KA, or metabotropic receptors; other ligand-gated ion channels which promote depolarization and/or toxin release; voltage gated calcium channels including those of the L-, P-, Q/R-, N-, or T- types; voltage gated sodium channels. Examples of compounds that inhibit these signaling pathways include, but are not limited to, AMPA/kainate receptor antagonists, sodium channel antagonists and calcium channel antagonists.

Another approach to inhibiting cellular depolarization caused by neurodegenerative diseases and the resultant deleterious effects is to activate signaling pathways that oppose those causing depolarization. Again, the beneficial effects of activating these signaling mechanisms may be additive or synergistic with the benefits of inhibiting nitric oxide synthase. These signaling mechanisms include, but are not limited to: GABA_{A} receptor activation; voltage or ligand gated potassium channel activation; voltage or ligand gated chloride channel activation. Examples of compounds that activate these signaling pathways include, but are not limited to, potassium channel openers and GABA-A receptor agonists.

Excessive cellular depolarization and the loss of ionic homeostasis can lead to the loss in the ability of a cell to maintain physical integrity and cellular death ensues by a process often termed necrotic cell death. However, neurodegenerative diseases can also induce in many cells the activation of another mechanism causing cellular death that is termed apoptosis. The relationship between necrotic and apoptotic cell death is not fully understood and in pathological conditions such as neurodegenerative diseases both necrotic and apoptotic mechanisms leading ultimately toward cell death may be at play. Regardless of the specifics of this interrelationship, it has been suggested that inhibition of apoptotic mechanism of cell death may have a therapeutic benefit in neurodegenerative diseases. The beneficial effects of inhibiting apoptosis during neurodegenerative diseases may be additive or synergistic with the benefits of inhibiting n nitric oxide synthase. Apoptotic mechanisms include, but are not limited to: activation of FAS/TNFα/p75 receptors; activation of caspases including caspases 1 through 9; activation of NFκB; activation of the JNK and/or p38 kinase signaling cascades; inhibition of mitochondrial disruption and the activation of the mitochondrial permeability transition pore; activation of intracellular proteases such as the calpains. Examples of compounds that inhibit these apoptotic mechanisms include, but are not limited to, caspase inhibitors and inhibitors of the other enzymes mentioned above as mediators of apoptotic mechanisms

Cells in the CNS are highly dependent on cell-to-cell interactions and interaction with the extracellular matrix for survival and proper function. However, during neurodegenerative diseases these interactions are often disrupted and this can lead directly to or contribute to cellular dysfunction and death. Thus, therapies that maintain cell-to-cell and cell-to-extracellular matrix interaction during ischemic, hypoxic or traumatic CNS insult are expected to reduce dysfunction and cell death. Furthermore, the beneficial effects of therapies that maintain cell-to-cell and cell-to-extracellular matrix interaction during neurodegenerative diseases may be additive or synergistic with the benefits of inhibiting an N-NOS antagonist. Mechanisms that contribute to the disruption of cell-to-cell and cell-to-extracellular matrix interaction during ischemic, hypoxic neurodegenerative diseases include, but are not limited to, the activation of proteases which degrade the extracellular matrix. These include, but are not limited to, matrix metalloproteases such as MMP 1 through 13. Examples of compounds that inhibit these enzymes include, but are not limited to those referred to in the following patents and patent applications: U.S. Patent 5,753,653 issued May 19, 1998; U.S. Patent 5,861,510, issued January 19, 1999; European Patent Application EP 606,046, published July 13, 1994; European Patent Application EP 935,963, published August 18, 1999; PCT Patent Application WO 98/34918, published August 13, 1998; PCT Patent Applications WO 98/08825 and WO 98/08815, both published March 5, 1998; PCT Patent Application WO 98/03516, published January 29, 1998; and PCT Patent Application WO 98/33768, published August 6, 1998. The foregoing patents and patent applications are incorporated herein by reference in their entireties.

Neurodegenerative diseases lead to an inflammatory response mediated by various components of the innate and adaptive immune system. Because of the nature of the CNS and its unique relationship to the immune system, the immune system activation caused by neurodegenerative diseases can exacerbate cellular dysfunction and death. The mechanisms whereby immune activation exacerbates CNS injury are many-fold. Immune cells resident to the CNS, such as astrocytes and microglia, are activated following CNS injury. Furthermore, peripheral immune cells are recruited to enter the CNS and also become activated. These cells include monocytes/macrophages, neutrophils, and T lymphopcytes. Recruitment and activation of these peripheral immune cells into the CNS after injury involves many of the same mechanisms by which these cells are recruited to and activated by injured tissue outside the CNS. The cell within the area of tissue injury and the vasculature around the site of injury begins to elaborate proteins that signal to immune cells circulating in the blood stream. These cells then adhere to the vascular epithelium and enter the area in and around the damaged tissue. These activated immune cells then promote many of the deleterious events listed above, including release of a variety of toxins and disruption of cell-to-cell and cell-to-extracellular matrix interactions.

Thus, inhibition of immune cell recruitment, adherence to the vasculature, activation, and formation and release of toxins and proteases in response to neurodegenerative disease is hypothesized to reduce the cellular dysfunction and death caused by these CNS insults. The beneficial effects of inhibiting immune cell recruitment, activation, and formation and release of toxins and proteases during ischemic, hypoxic or traumatic CNS injury may be additive or synergistic with the benefits of inhibiting neuronal nitric oxide synthase. Compounds that inhibit immune cell recruitment include, but are not limited to, non-steroidal anitiinflammatory agents such as piroxicam and celecoxib and also auranofin and methotrexate.

### Summary of the Invention

This invention relates to a method of treating neurodegenerative diseases selected from the group consisting of stroke, hypovolemic shock, traumatic shock, reperfusion injury, multiple sclerosis, AIDS, associated dementia; neuron toxicity, Alzheimers disease, head trauma, adult respiratory disease (ARDS), acute spiral cord injury, Huntington's disease, and Parkinson's Disease diseases in a mammal, including a human, comprising administering to said mammal:
(a) an N-NOS inhibitor or a pharmaceutically acceptable salt thereof; and
(b) a selective NMDA receptor antagonizing receptor compound or a pharmaceutically acceptable salt thereof;
wherein the active agents "a" and "b" above are present in amounts that render the combination of the two agents effective in treating neurodegenerative diseases.

This invention also relates to a pharmaceutical composition for treating neurodegenerative diseases selected from the group consisting of stroke, hypovolemic shock, traumatic shock, reparfusion injury, multiple sclerosis, AIDS, associated dementia, neuron toxicity, Alzheimer's disease, head trauma, aduct respiratory disease (ARDS), acute spiral cord injury, Huntington's disease, and Parkinson's Disease, in a mammal, including a human, comprising:
(a) an N-NOS inhibitor or a pharmaceutically acceptable salt thereof;
(b) a selective NMDA receptor antagonizing compound or a pharmaceutically acceptable salt thereof; and
c) a pharmaceutically acceptable carrier;
wherein the active agents "a" and "b" are present in such composition in amounts that render the combination of the two agents effective in treating such disorder.

Examples of NOS inhibiting compounds that can be used in the methods and pharmaceutical compositions of the present invention are those referred to in: U.S. provisional application 60/057094, which was filed August 27, 1997 and is entitled "2-Aminopyrindines Containing Fused Ring Substituents"; the PCT application having the same title that was filed on May 5, 1998, which designates the United States and claims priority from provisional application 60/057094; PCT patent application WO 97/36871, which designates the United States and was published on October 9, 1997; U.S. provisional patent application 60/057739 of John A. Lowe, III, entitled "6-Phenylpyridin-2-yl-amine Derivatives", which was filed on August 28, 1997; PCT patent application PCT/IB98/00112, entitled "4-Amino-6-(2-substituted-4-phenoxy)-substituted-pyridines", which designates the United States and was filed on January 29, 1998; PCT patent application PCT/IB97/01446, entitled "6-Phenylpyridyl-2-amine Derivatives", which designates the United States and was filed on November 17, 1997; and the U.S. provisional application of John A. Lowe, III, that was filed on June 3, 1998 and is entitled "2-Aminopyridines Containing Fused Ring Substituents". The foregoing patent applications are incorporated herein by reference in their entirety.

Preferred methods and pharmaceutical compositions include the above described methods and pharmaceutical compositions wherein the N- NOS inhibitor is of the formula I 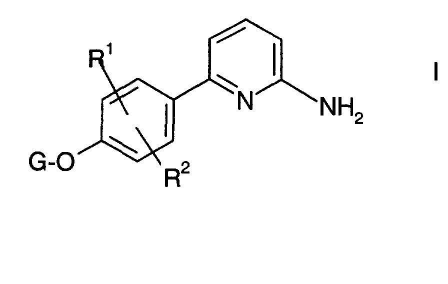 wherein R¹ and R² are selected, independently, from hydrogen, halo, hydroxy, (C₁-C₆)alkoxy, (C₁-C₇)alkyl, (C₂-C₆)alkenyl, and (C₂ - C₁₀)alkoxyalkyl; and

G is selected from hydrogen, (C₁-C₆)alkyl, (C₁-C₆)alkoxy-(C₁-C₃)alkyl, aminocarbonyl-(C₁-C₃)alkyl-, (C₁-C₃) alkylaminocarbonyl -(C₁-C₃) alkyl-, di-[(C₁-C₃)alkyl]aminocarbonyl-(C₁-C₃)alkyl-, and N(R³)(R⁴)(C₀-C₄)alkyl-, wherein R³ and R⁴ are selected, independently, from hydrogen, (C₁-C₇) alkyl, tetrahydronaphthalene and aralkyl, wherein the aryl moiety of said aralkyl is phenyl or naphthyl and the alkyl moiety is straight or branched and contains from 1 to 6 carbon atoms, and wherein said (C₁-C₇) alkyl and said tetrahydronaphthalene and the aryl moiety of said aralkyl may optionally be substituted with from one to three substituents, preferably from zero to two substituents, that are selected, independently, from halo, nitro, hydroxy, cyano, amino, (C₁-C₄) alkoxy, and (C₁-C₄) alkylamino;
or R³ and R⁴ form, together with the nitrogen to which they are attached, a piperazine, piperidine, azetidine or pyrrolidine ring or a saturated or unsaturated azabicyclic ring system containing from 6 to 14 ring members, from 1 to 3 of which are nitrogen, from zero to two of which are oxygen, and the rest of which are carbon;
and wherein said piperazine, piperidine, azetidine and pyrrolidine rings and said azabicyclic ring systems may optionally be substituted with one or more substituents, preferably with from zero to two substituents, that are selected, independently, from (C₁-C₆)alkyl, amino, (C₁-C₆) alkylamino, [di-(C₁-C₆)alkyl]amino, phenyl substituted 5 to 6 membered heterocyclic rings containing from 1 to 4 ring nitrogen atoms, benzoyl, benzoylmethyl, benzylcarbonyl, phenylaminocarbonyl, phenylethyl and phenoxycarbonyl, and wherein the phenyl moieties of any of the foregoing substituents may optionally be substituted with one or more substituents, preferably with from zero to two substituents, that are selected, independently, from halo, (C₁-C₃)alkyl, (C₁-C₃)alkoxy, nitro, amino, cyano, CF₃ and OCF₃;
and wherein said piperazine, piperidine, azetidine and pyrrolidine rings and said azabicyclic ring systems may be attached to -(C₀-C₄)alkyl-O- (wherein the oxygen of said -(C₀-C₄)alkyl-O- is the oxygen atom depicted in structural formula I) at a nitrogen atom of the NR³R⁴ ring or at any other atom of such ring having an available bonding site;
or G is a group of the formula A 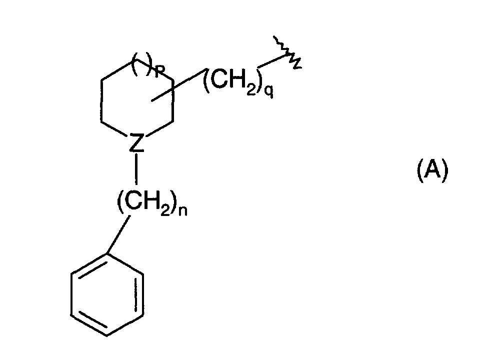 wherein Z is nitrogen or CH, n is zero or one, q is zero, one, two or three and p is zero, one or two;
and wherein the 2-amino piperidine ring depicted in structure I above may optionally be replaced with 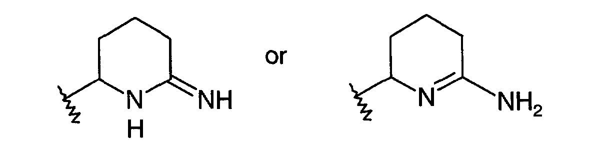 and the pharmaceutically acceptable salts of such compounds.

The present invention also relates to the pharmaceutically acceptable acid addition salts of compounds of the formula I. The acids which are used to prepare the pharmaceutically acceptable acid addition salts of the aforementioned base compounds of this invention are those which form non-toxic acid addition salts, i.e., salts containing pharmacologically acceptable anions, such as the hydrochloride, hydrobromide, hydroiodide, nitrate, sulfate, bisulfate, phosphate, acid phosphate, acetate, lactate, citrate, acid citrate, tartrate, bitartrate, succinate, maleate, fumarate, gluconate, saccharate, benzoate, methanesulfonate, ethanesulfonate, benzenesulfonate, p-toluenesulfonate and pamoate [i.e., 1,1-methylene-bis-(2-hydroxy-3-naphthoate)] salts.

The term "alkyl", as used herein, unless otherwise indicated, includes saturated monovalent hydrocarbon radicals having straight, branched or cyclic moieties or combinations thereof.

The term "one or more substituents", as used herein, refers to a number of substituents that equals from one to the maximum number of substituents possible based on the number of available bonding sites.

The term "halo", as used herein, unless otherwise indicated, includes chloro, fluoro, bromo and iodo.

Examples of compounds of this invention are compounds of the formula I, and their pharmaceutically acceptable salts, wherein G is N(R³)(R⁴)(C₀-C₄) alkyl and N(R³)(R⁴) is amino, dimethylamino, methylbenzylamino, (C₁-C₄)alkylamino, di-[(C₁-C₄)alkyllamino or one of the following groups:

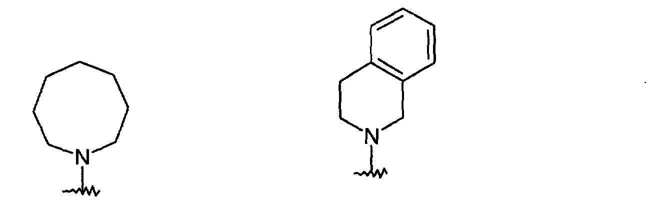

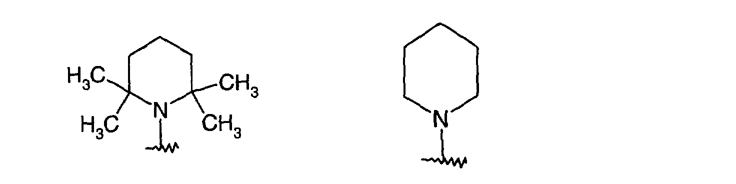

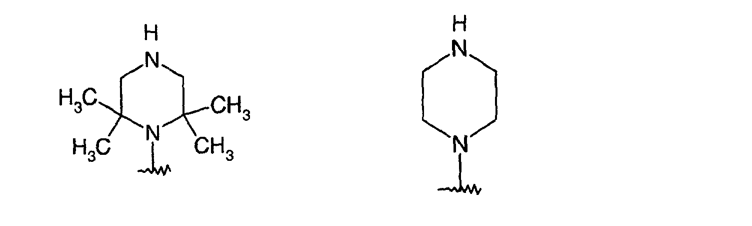

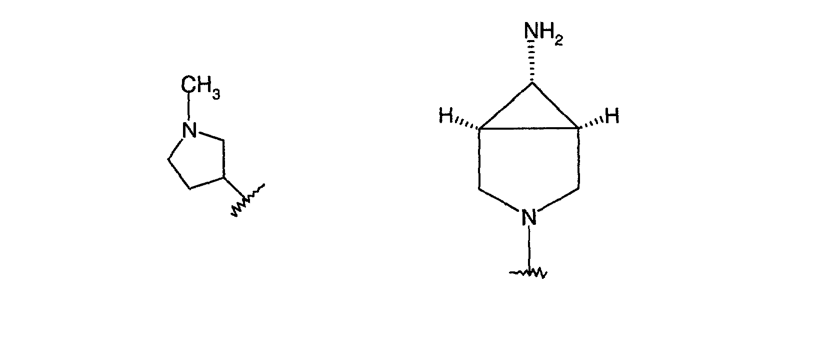

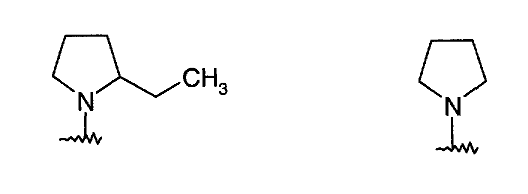

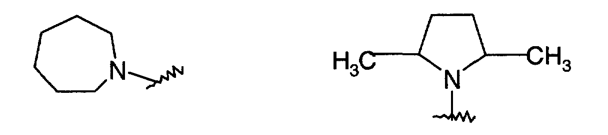

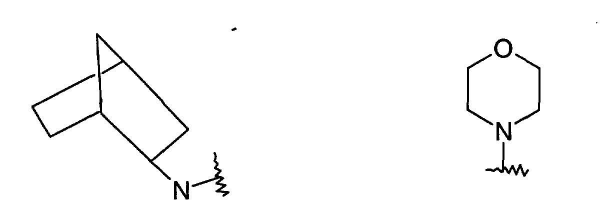

Preferred compounds of the formula I include those wherein R² is hydrogen and R¹ is (C₁ - C₃)alkoxy and is in the ortho position relative to the pyridine ring of formula I.

Other embodiments of this invention relate to compounds of the formula I wherein G is a group of the formula A, as defined above, wherein Z is nitrogen.

Other embodiments of this invention relate to compounds of the formula I wherein R¹ and R² are selected, independently, from (C₁-C₂)alkoxy.

Other embodiments of the invention relate to compounds of the formula I wherein G is a group of the formula A, as defined above, wherein Z is nitrogen, each of p and n is one and q is two.

Other embodiments of this invention relate to compounds of the formula I wherein the 2-aminopyridine ring depicted in formula I above, is present.

The term "treating", as used herein, refers to retarding or reversing the progress of, or alleviating or preventing either the disorder or condition to which the term "treating" applies, or one or more symptoms of such disorder or condition. The term "treatment", as used herein, refers to the act of treating a disorder or condition, as the term "treating" is defined above.

The methods and pharmaceutical compositions of this invention include the above described methods and pharmaceutical compositions wherein the NMDA receptor antagonist is a selective NMDA receptor antagonist of the formula II 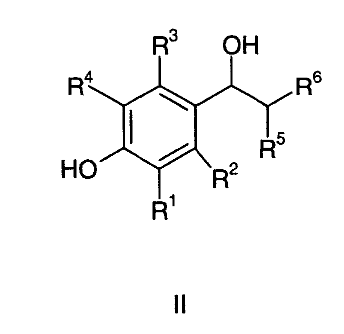 or a pharmaceutically acceptable acid addition salt thereof, wherein:
(a) R² and R⁵ are taken separately and R¹, R², R³ and R⁴ are each independently hydrogen, (C₁-C₆) alkyl, halo, CF₃, OH or OR⁷ and R⁵ is methyl or ethyl; or
(b) R² and R⁵ are taken together and are 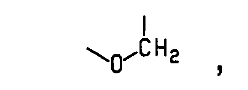 forming a chroman-4-ol ring, and R¹, R³ and R⁴ are each independently hydrogen, (C₁-C₆) alkyl, halo, CF₃, OH or OR⁷;
   R⁶ is 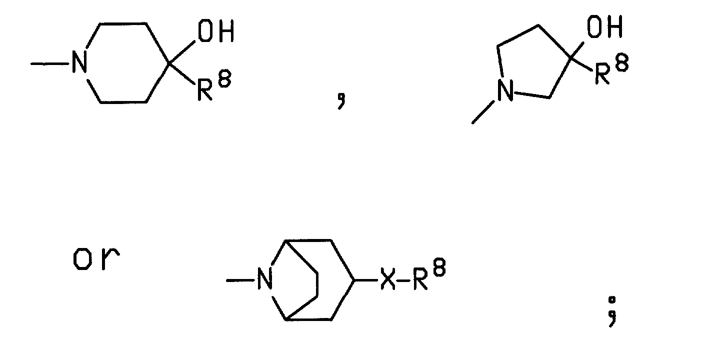
   R⁷ is methyl, ethyl, isopropyl or n-propyl;
   R⁸ is phenyl optionally substituted with up to three substituents independently selected from (C₁-C₆) alkyl, halo and CF₃;
   X is O, S or (CH₂)ₙ; and
   n is 0, 1, 2, or 3.

Compounds of formula II are described in United States Patents 5,185,343; 5,272,160; 5,338,754; 5,356,905; and 6,046,213 (which issued, respectively, on February 9, 1993, December 21, 1993, August 16, 1994, October 18, 1994, and April 4, 2000); United States Patent Applications Serial Numbers 08/292,651 (filed August 18, 1994), 08/189,479 (filed January 31, 1994) and 09/011,426 (filed June 20, 1996); PCT International Application No. PCT/IB95/00398, which designates the United States (filed May 26, 1995) (corresponding to WO 96/37222); and PCT International Application No. PCT/IB95/00380, which designates the United States (filed May 18, 1995) (corresponding to WO 96/06081). All of the foregoing patents, United States patent applications and PCT international application are herein incorporated by reference in their entirety.

Preferred compounds for use in the methods and pharmaceutical compositions of the present invention include those of formula I wherein R² and R⁵ are taken separately; R² and R³ are hydrogen; R⁶ is 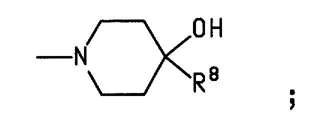 and R⁸ is phenyl, 4-halophenyl or 4-trifluoromethylphenyl. Within this group, more specific preferred compounds are those wherein R⁵ is methyl having a 1S*,2S* relative stereochemistry: 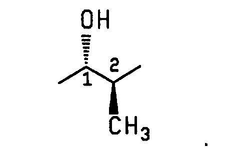 Other preferred compounds for use in the methods and pharmaceutical compositions of the present invention include those of formula I wherein R² and R⁵ are taken together and are 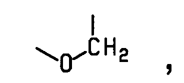 forming a chroman-4-ol ring. Within this group, preferred compounds also include those wherein the C-3 and C-4 positions of said chroman-4-ol ring have a 3R*,4S* relative stereochemistry: 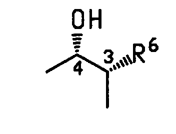 Within this group, preferred compounds also include those wherein R⁶ is 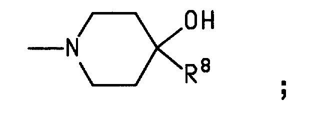 and R⁸ is phenyl or 4-halophenyl.

Compounds of formula II may contain chiral centers and therefore may exist in different enantiomeric and diastereomeric forms. This invention relates to the above methods of treatment using and the above pharmaceutical compositions comprising all optical isomers and all stereoisomers of compounds of the formula I and mixtures thereof.

The term "alkyl", as used herein, unless otherwise indicated, includes saturated monovalent hydrocarbon radicals having straight, branched or cyclic moieties or combinations thereof.

The term "one or more substituents", as used herein, refers to a number of substituents that equals from one to the maximum number of substituents possible based on the number of available bonding sites.

The terms "halo" and "halogen", as used herein, unless otherwise indicated, include chloro, fluoro, bromo and iodo.

Formula II above includes compounds identical to those depicted but for the fact that one or more hydrogen, carbon or other atoms are replaced by isotopes thereof. Such compounds may be useful as research and diagnostic tools in metabolism pharmacokinetic studies and in binding assays.

NMDA receptor antagonists of the formula II that are particularly preferred for use in the methods and pharmaceutical compositions of this invention are the following: (+)-(1S, 2S)-1-(4-hydroxy-phenyl)-2-(4-hydroxy-4-phenylpiperidino)-1-yl)-1-propanol; (1S,2S)-1-(4-hydroxy-3-methoxyphenyl)-2-(4-hydroxy-4-phenylpiperidino)-1 -propanol; (1S,2S)-1-(4-hydroxy-3-methyl phenyl)-2-hydroxy-4-phenyl (piperidino)-1-propanol and (3R,4S)-3-(4-(4-fluorophenyl)-4-hydroxypiperidin-1-yl)-chroman-4,7-diol.

This invention also relates to a method of treating neurodegenerative diseases selected from the group consisting of stroke, hypovolemic shock, traumatic shock, reperfusion injury, multiple sclerosis, AIDS, associated dementia; neuron toxicity, Alzheimers disease, head trauma, adult respiratory disease (ARDS), acute spiral cord injury, Huntington's disease, and Parkinson's Disease in a mammal, including a human, comprising administering to said mammal:
(a) an N-NOS inhibitor or a pharmaceutically acceptable salt thereof; and
(b) L-Dopa or a pharmaceutically acceptable salt thereof;
wherein the active agents "a" and "b" above are present in amounts that render the combination of the two agents effective in treating neurodegenerative diseases.

This invention also relates to a pharmaceutical composition for treating neurodegenerative diseases selected from the group consisting of stroke, hypovolemic shock, traumatic shock, reperfusion injury, multiple sclerosis, AIDS, associated dementia; neuron toxicity, Alzheimers disease, head trauma, adult respiratory disease (ARDS), acute spiral cord injury, Huntington's disease, and Parkinson's Disease in a mammal, including a human, comprising:
(a) an N-NOS inhibitor or a pharmaceutically acceptable salt thereof;
(b) L-Dopa or a pharmaceutically acceptable salt thereof;
(c) a pharmaceutically acceptable carrier wherein the active agents "a" and "b" above are present in such compositions in amounts that render the combination of the two agents effective in treating such disorder.

This invention also relates to a method of treating neurodegenerative diseases selected from the group consisting of stroke, hypovolemic shock, traumatic shock, reperfusion injury, multiple sclerosis, AIDS, associated dementia; neuron toxicity, Alzheimers disease, head trauma, adult respiratory disease (ARDS), acute spiral cord injury, Huntington's disease, and Parkinson's Disease in a mammal, including a human, comprising administering to said mammal:
(a) a sodium channel antagonist or a pharmaceutically acceptable salt thereof; and
(b) an N-NOS inhibitor or a pharmaceutically acceptable salt thereof;
wherein the active agents "a" and "b" above are present in amounts that render the combination of the two agents effective in treating neurogenerative diseases.

This invention also relates to a pharmaceutical composition for treating neurodegenerative diseases selected from the group consisting of stroke, hypovolemic shock, traumatic shock, reperfusion injury, multiple sclerosis, AIDS, associated dementia; neuron toxicity, Alzheimers disease, head trauma, adult respiratory disease (ARDS), acute spiral cord injury, Huntington's disease, and Parkinson's Disease in a mammal, including a human, comprising:
(a) a sodium channel antagonist or a pharmaceutically acceptable salt thereof;
(b) an N-NOS inhibitor or a pharmaceutically acceptable salt thereof; and
(c) a pharmaceutically acceptable carrier;
wherein the active agents "a" and "b" are present in such composition in amounts that render the combination of the two agents effective in treating such disorder.

Examples of suitable sodium channel blocking compounds *(i.e.,* sodium channel antagonists) that can be employed in the methods and pharmaceutical compositions of this invention, as described above, are ajmaline, procainamide, flecainide and riluzole.

This invention also relates to a method of treating neurodegenerative diseases selected from the group consisting of stroke, hypovolemic shock, traumatic shock, reperfusion injury, multiple sclerosis, AIDS, associated dementia; neuron toxicity, alzheimers disease, head trauma, adult respiratory disease (ARDS), acute spiral cord injury, Huntington's disease, and Parkinson's Disease in a mammal, including a human, comprising administering to said mammal:
(a) a calcium channel antagonist or a pharmaceutically acceptable salt thereof; and
(b) an N-NOS inhibiting agent or a pharmaceutically acceptable salt thereof;
wherein the active agents "a" and "b" above are present in amounts that render the combination of the two agents effective in treating neurodegenerative diseases.

This invention also relates to a pharmaceutical composition for treating neurodegenerative diseases selected from the group consisting of stroke, hypovolemic shock, traumatic shock, reperfusion injury, multiple sclerosis, AIDS, associated dementia; neuron toxicity, Alzheimers disease, head trauma, adult respiratory disease (ARDS), acute spiral cord injury, Huntington's disease, and Parkinson's Disease in a mammal, including a human, comprising:
(a) a calcium channel antagonist or a pharmaceutically acceptable salt thereof;
(b) an N-NOS inhibiting agent or a pharmaceutically acceptable salt thereof; and
(c) a pharmaceutically acceptable carrier;
wherein the active agents "a" and "b" are present in such composition in amounts that render the combination of the two agents effective in treating such disorder.

Examples of suitable calcium channel blocking compounds (*i.e*., calcium channel antagonists) that can be employed in the methods and pharmaceutical compositions of this invention, as described above, are diltiazem, omega-conotoxin GVIA, methoxyverapamil, amlodipine, felodipine, lacidipine, and mibefradil.

This invention also relates to a method of treating neurodegenerative diseases selected from the group consisting of stroke, hypovolemic shock, traumatic shock, reperfusion injury, multiple sclerosis, AIDS, associated dementia; neuron toxicity, Alzheimers disease, head trauma, adult respiratory disease (ARDS), acute spiral cord injury, Huntington's disease, and Parkinson's Disease in a mammal, including a human, comprising administering to said mammal:
(a) a potassium channel opening compound or a pharmaceutically acceptable salt thereof; and
(b) an N-NOS inhibiting agent or a pharmaceutically acceptable salt thereof;
wherein the active agents "a" and "b" above are present in amounts that render the combination of the two agents effective in treating neurodegenerative diseases.

This invention also relates to a pharmaceutical composition for treating neurodegenerative diseases selected from the group consisting of stroke, hypovolemic shock, traumatic shock, reperfusion injury, multiple sclerosis, AIDS, associated dementia; neuron toxicity, Alzheimers disease, head trauma, adult respiratory disease (ARDS), acute spiral cord injury, Huntington's disease, and Parkinson's Disease in a mammal, including a human, comprising:
(a) a potassium channel opening compound or a pharmaceutically acceptable salt thereof;
(b) an N-NOS inhibiting agent or a pharmaceutically acceptable salt thereof; and
(c) a pharmaceutically acceptable carrier;
wherein the active agents "a" and "b" are present in such composition in amounts that render the combination of the two agents effective in treating such disorder.

Examples of suitable potassium channel openers that can be employed in the methods and pharmaceutical compositions of this invention, as described above, are diazoxide, flupirtine, pinacidil, levcromakalim, rilmakalim, chromakalim, PCO-400 (J. Vasc. Res., Nov. - Dec. 1999, 36 (6), 516-23) and SKP-450 (2-[2"(1", 3"-dioxolone)-2-methyl]-4-(2'-oxo-1 '-pyrrolidinyl)-6-nitro-2H-1-benzopyran).

This invention also relates to a method of treating neurodegenerative diseases selected from the group consisting of stroke, hypovolemic shock, traumatic shock, reperfusion injury, multiple sclerosis, AIDS, associated dementia; neuron toxicity, Alzheimers disease, head trauma, adult respiratory disease (ARDS), acute spiral cord injury, Huntington's disease, and Parkinson's Disease in a mammal, including a human, comprising administering to said mammal:
(a) a dopamine agonist or a pharmaceutically acceptable salt thereof; and
(b) an N-NOS inhibiting agent or a pharmaceutically acceptable salt thereof;
wherein the active agents "a" and "b" above are present in amounts that render the combination of the two agents effective in treating neurodegenerative diseases.

This invention also relates to a pharmaceutical composition for treating neurodegenerative diseases selected from the group consisting of stroke, hypovolemic shock, traumatic shock, reperfusion injury, multiple sclerosis, AIDS, associated dementia; neuron toxicity, Alzheimers disease, head trauma, adult respiratory disease (ARDS), acute spiral cord injury, Huntington's disease, and Parkinson's Disease in a mammal, including a human, comprising:
(a) a dopamine agonist or a pharmaceutically acceptable salt thereof;
(b) an N-NOS inhibiting agent or a pharmaceutically acceptable salt thereof; and
c) a pharmaceutically acceptable carrier;
wherein the active agents "a" and "b" are present in such composition in amounts that render the combination of the two agents effective in treating such disorder.

Examples of suitable dopamine agonists that can be employed in the methods and pharmaceutical compositions of this invention, as described above, are ropinole. L-dopa in combination with an L-dopa decarboxylase inhibitor such as carbidopa or benserazide, bromocriptine, dihydroergocryptine, etisulergine, AF-14, alaptide, pergolide, piribedil, dopamine D1 receptor agonists such as A-68939, A-77636, dihydrexine, and SKF-38393; dopamine D2 receptor agonists such as carbergoline, lisuride, N-0434, naxagolide, PD-118440, pramipexole, quinpirole and ropinirole; dopamine/β-adrenergic receptor agonists such as DPDMS and dopexamine; dopamine/5-HT uptake inhibitor/5-HT-1A agonists such as roxindole; dopamine/opiate receptor agonists such as NIH-10494; α2-adrenergic antagonist/dopamine agonists such as terguride; α2-adrenergic antagonist/dopamine D2 agonists such as ergolines and talipexole; dopamine uptake inhibitors such as GBR-12909, GBR-13069, GYKI-52895, and NS-2141; monoamine oxidase-B inhibitors such as selegiline, N-(2-butyl)-N-methylpropargylamine, N-methyl-N-(2-pentyl)propargylamine, AGN-1133, ergot derivatives, lazabemide, LU-53439, MD-280040 and mofegiline; and COMT inhibitors such as CGP-28014,

This invention also relates to a method of treating neurodegenerative diseases selected from the group consisting of stroke, hypovolemic shock, traumatic shock, reperfusion injury, multiple sclerosis, AIDS, associated dementia; neuron toxicity, Alzheimers disease, head trauma, adult respiratory disease (ARDS), acute spiral cord injury, Huntington's disease, and Parkinson's Disease in a mammal, including a human, comprising administering to said mammal:
(a) a GABA-A receptor modulator (*e.g.,* a GABA-A receptor agonist) or a pharmaceutically acceptable salt thereof; and
(b) an N-NOS inhibiting agent or a pharmaceutically acceptable salt thereof;
wherein the active agents "a" and "b" above are present in amounts that render the combination of the two agents effective in treating neurodegenerative disease.

This invention also relates to a pharmaceutical composition for treating neurodegenerative diseases selected from the group consisting of stroke, hypovolemic shock, traumatic shock, reperfusion injury, multiple sclerosis, AIDS, associated dementia; neuron toxicity, Alzheimers disease, head trauma, adult respiratory disease (ARDS), acute spiral cord injury, Huntington's disease, and Parkinson's Disease in a mammal, including a human, comprising:
(a) a GABA-A receptor modulator (*e.g.,* a GABA-A receptor agonist) or a pharmaceutically acceptable salt thereof;
(b) an N-NOS inhibiting agent a pharmaceutically acceptable salt thereof; and
c) a pharmaceutically acceptable carrier;
wherein the active agents "a" and "b" are present in such composition in amounts that render the combination of the two agents effective in treating such disorder.

Examples of suitable GABA-A receptor modulators that can be employed in the methods and pharmaceutical compositions of this invention, as described above, are clomethiazole; IDDB; gaboxadol (4,5,6,7-tetrahydroisoxazolo[5,4-c]pyridin-3-ol); ganaxolone (3α-hydroxy-3β-methyl-5α-pregnan-20-one); fengabine(2-[(butylimino)-(2-chlorophenyl) methyl]-4-chlorophenol); 2-(4-methoxyphenyl)-2,5,6,7,8,9-hexahydro-pyrazolo[4,3-c]cinnolin-3-one; 7-cyclobutyl-6-(2-methyl-2H-1,2,4-triazol-3-ylmethoxy)-3-phenyl-1,2,4-triazolo[4,3-b]pyridazine; (3-fluoro-4-methylphenyl)-N-({1-[(2-methylphenyl)methyl]-benzimidazol-2-yl}methyl)-N-pentylcarboxamide; and 3-(aminomethyl)-5-methylhexanoic acid.

Other examples of GABA-A modulators that can be used in the pharmaceutical compositions and methods of this invention are those that are referred to in the following: World Patent Application WO 99/25353, which was published on May 27, 1999; World Patent Application WO 96/25948, which was published on August 29, 1996; World Patent Application WO 99/37303, which was published on July 29, 1999; United States Patent 5,925,770, which was issued on July 20, 1999; United States Patent 5,216,159, which was issued on June 1, 1993; United States Patent 5,130,430, which was issued on July 14, 1992; United States Patent 5,925,770, which was issued on July 20, 1999; and World Patent Application WO 99/10347, which was published on March 4, 1999.

This invention also relates to a method of treating neurodegenerative diseases selected from the group consisting of stroke, hypovolemic shock, traumatic shock, reperfusion injury, multiple sclerosis, AIDS, associated dementia; neuron toxicity, Alzheimers disease, head trauma, adult respiratory disease (ARDS), acute spiral cord injury, Huntington's disease, and Parkinson's Disease in a mammal, including a human, comprising administering to said mammal:
(a) TPA or a pharmaceutically acceptable salt thereof; and
(b) an N-NOS Inhibitor or a pharmaceutically acceptable salt thereof;
wherein the active agents "a" and "b" above are present in amounts that render the combination of the two agents effective in treating neurodegenerative diseases.

This invention also relates to a pharmaceutical composition for treating neurodegenerative diseases selected from the group consisting of stroke, hypovolemic shock, traumatic shock, reperfusion injury, multiple sclerosis, AIDS, associated dementia; neuron toxicity, Alzheimers disease, head trauma, adult respiratory disease (ARDS), acute spiral cord injury, Huntington's disease, and Parkinson's Disease in a mammal, including a human, comprising:
(a) an N-NOS inhibiting agent or a pharmaceutically acceptable salt thereof;
(b) TPA or a pharmaceutically acceptable salt thereof; and
(c) a pharmaceutically acceptable carrier;
wherein the active agents "a" and "b" are present in such composition in amounts that render the combination of the two agents effective in treating such disorder.

This invention also relates to a method of treating neurodegenerative diseases selected from the group consisting of stroke, hypovolemic shock, traumatic shock, reperfusion injury, multiple sclerosis, AIDS, associated dementia; neuron toxicity, Alzheimers disease, head trauma, adult respiratory disease (ARDS), acute spiral cord injury, Huntington's disease, and Parkinson's Disease in a mammal, including a human, comprising administering to said mammal:
(a) an AMPA/kainate receptor antagonizing compound or a pharmaceutically acceptable salt thereof; and
(b) an N-NOS inhibiting agent or a pharmaceutically acceptable salt thereof;
wherein the active agents "a" and "b" above are present in amounts that render the combination of the two agents effective in treating neurodegenerative diseases.

This invention also relates to a pharmaceutical composition for treating neurodegenerative diseases selected from the group consisting of stroke, hypovolemic shock, traumatic shock, reperfusion injury, multiple sclerosis, AIDS, associated dementia; neuron toxicity, Alzheimers disease, head trauma, adult respiratory disease (ARDS), acute spiral cord injury, Huntington's disease, and Parkinson's Disease in a mammal, including a human, comprising:
(a) an AMPA/kainate receptor antagonizing compound or a pharmaceutically acceptable salt thereof;
(b) an N-NOS inhibiting agent or a pharmaceutically acceptable salt thereof; and
(c) a pharmaceutically acceptable carrier;
wherein the active agents "a" and "b" are present in such composition in amounts that render the combination of the two agents effective in treating such disorder.

Examples of suitable AMPA/kainate receptor antagonizing compounds that can be employed in the methods and pharmaceutical compositions of this invention, as described above, are 6-cyano-7-nitroquinoxalin-2,3-dione (CNQX); 6-nitro-7-sulphamoylbenzo[f]quinoxaline-2,3-dione (NBQX); 6,7-dinitroquinoxaline-2,3-dione (DNQX); 1-(4-aminophenyl)-4-methyl-7,8-methylenedioxy-5H-2,3-benzodiazepine hydrochloride; and 2,3-dihydroxy-6-nitro-7-sulfamoylbenzo-[f]quinoxaline.

This invention also relates to a method of treating neurodegenerative diseases selected from the group consisting of stroke, hypovolemic shock, traumatic shock, reperfusion injury, multiple sclerosis, AIDS, associated dementia; neuron toxicity, Alzheimers disease, head trauma, adult respiratory disease (ARDS), acute spiral cord injury, Huntington's disease, and Parkinson's Disease in a mammal, including a human, comprising administering to said mammal:
(a) a matrix-metalloprotease inhibitor or a pharmaceutically acceptable salt thereof; and
(b) an N-NOS inhibiting agent or a pharmaceutically acceptable salt thereof;
wherein the active agents "a" and "b" above are present in amounts that render the combination of the two agents effective in treating neurodegenerative diseases.

This invention also relates to a pharmaceutical composition for treating neurodegenerative diseases selected from the group consisting of stroke, hypovolemic shock, traumatic shock, reperfusion injury, multiple sclerosis, AIDS, associated dementia; neuron toxicity, Alzheimers disease, head trauma, adult respiratory disease (ARDS), acute spiral cord injury, Huntington's disease, and Parkinson's Disease in a mammal, including a human, comprising:
(a) a matrix-metalloprotease inhibitor or a pharmaceutically acceptable salt thereof;
(b) an N-NOS inhibiting agent or a pharmaceutically acceptable salt thereof; and
(c) a pharmaceutically acceptable carrier;
wherein the active agents "a" and "b" are present in such composition in amounts that render the combination of the two agents effective in treating such disorder.

Examples of suitable matrix-metalloprotease inhibitors that can be employed in the methods and pharmaceutical compositions of this invention, as described above, are
4-[4-(4-fluorophenoxy)benzenesulfonylamino]tetrahydropyran-4-carboxylic acid hydroxyamide;
5-Methyl-5-(4-(4'-fluorophenoxy)-phenoxy)-pyrimidine-2,4,6-trione;
5-n-Butyl-5-(4-(4'-fluorophenoxy)-phenoxy)-pyrimidine-2,4,6-trione; and prinomistat.

This invention also relates to a method of treating neurodegenerative diseases selected from the group consisting of stroke, hypovolemic shock, traumatic shock, reperfusion injury, multiple sclerosis, AIDS, associated dementia; neuron toxicity, Alzheimers disease, head trauma, adult respiratory disease (ARDS), acute spiral cord injury, Huntington's disease, and Parkinson's Disease in a mammal, including a human, comprising administering to said mammal:
(a) a acetylcholine esterase inhibitors or a pharmaceutically acceptable salt thereof; and
(b) an N-NOS inhibiting agent or a pharmaceutically acceptable salt thereof;
wherein the active agents "a" and "b" above are present in amounts that render the combination of the two agents effective in treating neurodegenerative diseases.

This invention also relates to a pharmaceutical composition for treating neurodegenerative diseases selected from the group consisting of stroke, hypovolemic shock, traumatic shock, reperfusion injury, multiple sclerosis, AIDS, associated dementia; neuron toxicity, Alzheimers disease, head trauma, adult respiratory disease (ARDS), acute spiral cord injury, Huntington's disease, and Parkinson's Disease in a mammal, including a human, comprising:
(a) a acetylcholine esterase inhibitors or a pharmaceutically acceptable salt thereof;
(b) an N-NOS inhibiting agent or a pharmaceutically acceptable salt thereof; and
(c) a pharmaceutically acceptable carrier;
wherein the active agents "a" and "b" are present in such composition in amounts that render the combination of the two agents effective in treating such disorder.

Examples of suitable acetylcholine esterase inhibitors that can be employed in the methods and pharmaceutical compositions of this invention, as described above, are donepizil
1-(2-methyl-1H-benzimidazol-5-yl)-3-[1-(phenylmethyl)-4-piperidinyl]-1-propanone;
1-(2-phenyl-1H-benzimidazol-5-yl)-3-[1-(phenylmethyl)-4-piperidinyl]-1 -propanone;
1-(1-ethyl-2-methyl-1H-benzimidazol-5-yl)-3-[1 -(phenylmethyl)-4-piperidinyl]-1-propanone;
1 -(2-methyl-6-benzothiazolyl)-3-[1 -(phenylmethyl)-4-piperidinyl]-1 -propanone;
1 -(2-methyl-6-benzothiazolyl)-3-[1 -[(2-methyl-4-thiazolyl)methyl]-4-piperidinyl]-1-propanone;
1 -(5-methyl-benzo[b]thien-2-yl)-3-[1 -(phenylmethyl)-4-piperidinyl]-1 -propanone;
1-(6-methyl-benzo[b]thien-2-yl)-3-[1-(phenylmethyl)-4-piperidinyl]-1-propanone;
1-(3,5-dimethyl-benzo[b]thien-2-yl)-3-[1-(phenylmethyl)-4-piperidinyl]-1-propanone;
1-(benzo[b]thien-2-yl)-3-[1-(phenylmethyl)-4-piperidinyl]-1 -propanone;
1-(benzofuran-2-yl)-3-[1 -(phenylmethyl)-4-piperidinyl]-1-propanone;
1-(1-phenylsulfonyl-6-methyl-indol-2-yl)-3-[1-(phenylmethyl)-4-piperidinyl]-1-propanone;
1-(6-methyl-indol-2-yl)-3-[1 -(phenylmethyl)-4-piperidinyl]-1-propanone;
1-(1-phenylsulfonyl-5-amino-indol-2-yl)-3-[1-(phenylmethyl)-4-piperidinyl]-1 -propanone;
1-(5-amino-indol-2-yl)-3-[1-(phenylmethyl)-4-piperidinyl]-1-propanone; and
1-(5-acetylamino-indol-2-yl)-3-[1 -(phenylmethyl)-4-piperidinyl]-1 -propanone.
1-(6-quinolyl)-3-[1-(phenylmethyl)-4-piperidinyl]-1 -propanone;
1 -(5-indolyl)-3-[1 -(phenylmethyl)-4-piperidinyl]-1 -propanone;
1-(5-benzthienyl)-3-[1-(phenylmethyl)-4-piperidinyl]-1-propanone;
1 -(6-quinazolyl)-3-[1 -(phenylmethyl)-4-piperidinyl]-1 -propanone;
1 -(6-benzoxazolyl)-3-[1 -(phenylmethyl)-4-piperidinyl]-1-propanone;
1 -(5-benzofuranyl)-3-[1 -(phenylmethyl)-4-piperidinyl]-1-propanone;
1-(5-methyl-benzimidazol-2-yl)-3-[1-(phenylmethyl)-4-piperidinyl]-1 -propanone;
1 -(6-methyl-benzimidazol-2-yl)-3-[1 -(phenylmethyl)-4-piperidinyl]-1-propanone;
1 -(5-chloro-benzo[b]thien-2-yl)-3-[1 -(phenylmethyl)-4-piperidinyl]-1-propanone;
1-(5-azaindol-2-yl)-3-[1-(phenylmethyl)-4-piperidinyl]-1-propanone;
1 -(6-azabenzo[b]thien-2-yl)-3-[1-(phenylmethyl)-4-piperidinyl]-1-propanone;
1-(1H-2-oxo-pyrrolo[2N,3N,5,6]benzo[b]thieno-2-yl)-3-[1-(phenylmethyl)-4-piperidinyl]-1-propanone;
1-(6-methyl-benzothiazol-2-yl)-3-[1-(phenylmethyl)-4-piperidinyl]-1-propanone;
1-(6-methoxy-indol-2-yl)-3-[1-(phenylmethyl)-4-piperidinyl]-1-propanone;
1-(6-methoxy-benzo[b]then-2-yl)-3-[1-(phenylmethyl)-4-piperidinyl]-1-propanone;
1-(6-acetylamino-benzo[b]thien-2-yl)-3-[1 -(phenylmethyl)-4-piperidinyl]-1-propanone;
1-(5-acetylamino-benzo[b]thien-2-yl)-3-[1 -(phenylmethyl)-4-piperidinyl]-1-propanone;
6-hydroxy-3-[2-[1-(phenylmethyl)-4-piperidinyl]ethyl]-1,2-benzisoxazole;
5-methyl-3-[2-[1-(phenylmethyl)-4-piperidinyl]ethyl]-1,2-benzisoxazole;
6-methoxy-3[2-[-1 (phenylmethyl)-4-piperidinyl]ethyl]-1,2-benzisoxazole;
6-acetamido-3-[2-[1-(phenylmethyl)-4-piperidinyl]-ethyl]-1,2-benzisoxazole;
6-amino-3-[2-[1-(phenylmethyl)-4-piperidinyl]ethyl]-1,2-benzisoxazole;
6-(4-morpholinyl)-3-[2-[1-(phenylmethyl)-4-piperidinyl]ethyl]-1,2-benzisoxazole;
5,7-dihydro-3-[2-[1-(phenylmethyl)-4-piperidinyl]ethyl]-6H-pyrrolo[4,5-f]-1,2-benzisoxazol-6-one;
3-[2-[1-(phenylmethyl)-4-piperidinyl]ethyl]-1,2-benzisothiazole;
3-[2-[1-(phenylmethyl)-4-piperidinyl]ethenyl]-1,2-benzisoxazole;
6-phenylamino-3-[2-[1-(phenylmethyl)-4-piperidinyl]ethyl]-1,2,-benzisoxazole;
6-(2-thiazoly)-3-[2-[1-(phenylmethyl)-4-piperidinyl]ethyl]-1,2-benzisoxazole;
6-(2-oxazolyl)-3-[2-[1-(phenylmethyl)-4-piperidinyl]ethyl]-1,2-benzisoxazole;
6-pyrrolidinyl-3-[2-[1-(phenylmethyl)-4-piperidinyl]ethyl]-1,2-benzisoxazole;
5,7-dihydro-5,5-dimethyl-3-[2-[1-(phenylmethyl)-4-piperidinyl]ethyl]-6H-pyrrolo[4,5-f]-1,2-benzisoxazole-6-one;
6,8-dihydro-3-[2-[1-(phenylmethyl)-4-piperidinyl]ethyl]-7H-pyrrolo[5,4-g]-1,2-benzisoxazole-7-one;
3-[2-(1-(phenylmethyl)-4-piperidinyl]ethyl]-5,6,8-trihydro-7H-isoxazolo[4,5-g]-quinolin-7-one;
1-benzyl-4-((5,6-dimethoxy-1-indanon)-2-yl)methylpiperidine,
1-benzyl-4-((5,6-dimethoxy-1-indanon)-2-ylidenyl)methylpiperidine,
1-benzyl-4-((5-methoxy-1-indanon)-2-yl)methylpiperidine,
1-benzyl-4-((5,6-diethoxy-1-indanon)-2-yl)methylpiperidine,
1-benzyl-4-((5,6-methnylenedioxy-1-indanon)-2-yl)methylpiperidine,
1-(m-nitrobenzyl)-4-((5,6-dimethoxy-1-indanon)-2-yl)methylpiperidine,
1-cyclohexymethyl-4-((5,6-dimethoxy-1-indanon)-2-yl)methylpiperidine,
1-(m-florobenzyl)-4-((5,6-dimethoxy-1-indanon)-2-yl)methylpiperidine,
1-benzyl-4-((5,6-dimethoxy-1-indanon)-2-yl)propylpiperidine, and
1-benzyl-4-((5-isopropoxy-6-methoxy-1-indanon)-2-yl)methylpiperidine.

### Detailed Description of the Invention

Examples of N-NOS inhibiting compounds that can be used in the methods and pharmaceutical compositions of the present invention are those referred to in: U.S. provisional application 60/057094, which was filed August 27, 1997 and is entitled "2-Aminopyrindines Containing Fused Ring Substituents"; the PCT application having the same title that was filed on May 5, 1998, which designates the United States and claims priority from provisional application 60/057094; PCT patent application WO 97/36871, which designates the United States and was published on October 9, 1997; U.S. provisional patent application 60/057739 of John A. Lowe, III, entitled "6-Phenylpyridin-2-yl-amine Derivatives", which was filed on August 28, 1997; PCT patent application PCT/IB98/00112, entitled "4-Amino-6-(2-substituted-4-phenoxy)-substituted-pyridines", which designates the United States and was filed on January 29, 1998; PCT patent application PCT/IB97/01446, entitled "6-Phenylpyridyl-2-amine Derivatives", which designates the United States and was filed on November 17, 1997; and the U.S. provisional application of John A. Lowe, III, that was filed on June 3, 1998 and is entitled "2-Aminopyridines Containing Fused Ring Substituents". The foregoing patent applications are incorporated herein by reference in their entirety.

The NMDA antagonists of formula II are readily prepared. The compounds of formula II wherein R² and R⁵ are taken together forming a chroman-4-ol ring and R¹, R³, and R⁴ are hydrogen, can be prepared by one or more of the synthetic methods described in United States Patent 5,356,905, referred to above. The compounds of formula I wherein R² and R⁵ are taken separately and R¹, R², R³ and R⁴ are hydrogen can be prepared by one or more of the synthetic methods described in United States Patents 5,185,343, 5,272,160, and 5,338,754, all of which are referred to above. The compounds of formula I can also be prepared by one or more of the synthetic methods described in United States patent application serial numbers 08/292,651, 08/189,479 and 09/011,426; PCT International Application No. PCT/IB95/00398, which designates the United States (filed May 26, 1995) (corresponding to WO 96/37222); and PCT Application No. PCT/IB95/00380, which designates the United States (filed May 18, 1995) (corresponding to WO 96/06081), all of which are referred to above.

This invention relates both to methods of treatment in which the N-NOS inhibitor and the other active ingredient in the claimed combinations are administered together, as part of the same pharmaceutical composition, as well as to methods in which the two active agents are administered separately, as part of an appropriate dose regimen designed to obtain the benefits of the combination therapy. The appropriate dose regimen, the amount of each dose administered, and the intervals between doses of the active agents will depend upon the particular N-NOS inhibitors agent and other active ingredient being used in combination, the type of pharmaceutical formulation being used, the characteristics of the subject being treated and the severity of the disorder being treated.

Generally, in carrying out the methods of this invention, the dopamine antagonists will administered to an average adult human in amounts ranging from about 5 to about 300 mg per day, depending on the dopamine antagonists, severity of the condition and the route of administration. The acetyl cholinesterase inhibitors, in carrying out the methods of this invention, will generally be administered to an average adult human in amounts ranging from about 7 to about 2,000 mg per day. NMDA receptor antagonists, including glycine site antagonists, in carrying out the methods of this invention, will generally be administered to an average adult human in amounts ranging from about 25 to about 1500 mg per day. The AMPA/Kainate receptor antagonists will generally be administered to an average adult in amounts ranging from about 0.01 to 10 mg/kg body weight/per day.

The matrix-metalloprotease inhibitors, in carrying out the methods of this invention, will generally be administered to an average adult human in amounts ranging from about 0.1 to about 140 mg/kg body weight/per day.

The L-Dopa type compounds, in carrying out the methods of this invention, will generally be administered to an average adult human in amounts ranging from about 0.01 to about 10 mg/kg body weight/per day.

The TPA compounds, in carrying out the methods of this invention, will generally be administered to an average adult human in amounts ranging from about 0.001 to about 1 mg/kg body weight/per day.

The N-NOS inhibitor, in carrying out the methods of this invention, will generally be administered to an average adult human in amounts ranging from about 0.1 to about 100 mg/kg body weight/per day.

The GABA-A receptor modulators, calcium channel antagonists, potassium channel openers, sodium channel antagonists, in carrying out the methods of this invention, will generally be administered to an average adult human in amounts within the ranges used when such agents are administered, respectively, as single active pharmaceutical agents. Such dosages are available in the scientific and medical literature, and, for substances that have been approved for human use by the Food and Drug Administration, in the current edition (presently the 55^{rd} edition) of the Physician's Desk Reference, Medical Economics Company, Montvale, N.J.

In some instances, dosage levels below the lower limit of the aforesaid range may be more than adequate, while in other cases still larger doses may be employed without causing any harmful side effects, provided that such higher dose levels are first divided into several small doses for administration throughout the day.

The pharmaceutically active agents used in the methods and pharmaceutical compositions of this invention can be administered orally, parenterally, or topically, alone or in combination with pharmaceutically acceptable carriers or diluents, and such administration may be carried out in single or multiple doses. More particularly, the therapeutic agents of this invention can be administered in a wide variety of different dosage forms, i.e., they may be combined with various pharmaceutically acceptable inert carriers in the form of tablets, capsules, lozenges, troches, hard candies, powders, sprays, creams, salves, suppositories, jellies, gels, pastes, lotions, ointments, aqueous suspensions, injectable solutions, elixirs, syrups, and the like. Such carriers include solid diluents or fillers, sterile aqueous media and various non-toxic organic solvents, etc. Moreover, oral pharmaceutical compositions can be suitably sweetened and/or flavored. In general, the therapeutically-effective compounds of this invention are present in such dosage forms at concentration levels ranging from about 5.0% to about 70% by weight.

For oral administration, tablets containing various excipients such as microcrystalline cellulose, sodium citrate, calcium carbonate, dicalcium phosphate and glycine may be employed along with various disintegrants such as starch (and preferably corn, potato or tapioca starch), alginic acid and certain complex silicates, together with granulation binders like polyvinylpyrrolidone, sucrose, gelatin and acacia. Additionally, lubricating agents such as magnesium stearate, sodium lauryl sulfate and talc are often very useful for tabletting purposes. Solid compositions of a similar type may also be employed as fillers in gelatin capsules; preferred materials in this connection also include lactose or milk sugar as well as high molecular weight polyethylene glycols. When aqueous suspensions and/or elixirs are desired for oral administration, the active ingredient may be combined with various sweetening or flavoring agents, coloring matter or dyes, and, if so desired, emulsifying and/or suspending agents as well, together with such diluents as water, ethanol, propylene glycol, glycerin and various like combinations thereof.

For parenteral administration, solutions of a pharmaceutically active agent used in accordance with this invention in either sesame or peanut oil or in aqueous propylene glycol may be employed. The aqueous solutions should be suitably buffered (preferably pH greater than 8) if necessary and the liquid diluent first rendered isotonic. These aqueous solutions are suitable for intravenous injection purposes. The oily solutions are suitable for intra-articular, intra-muscular and subcutaneous injection purposes. The preparation of all these solutions under sterile conditions is readily accomplished by standard pharmaceutical techniques well known to those skilled in the art.

Additionally, it is also possible to administer the active agents used in accordance with the present invention topically, and this may be done by way of creams, jellies, gels, pastes, patches, ointments and the like, in accordance with standard pharmaceutical practice.

## Claims

1. A pharmaceutical composition for treating neurodegenerative diseases in a mammal, said composition comprising an N-NOS inhibitor or a pharmaceutically acceptable salt thereof in combination with at least one of:
- L-Dopa or a pharmaceutically acceptable salt thereof,
- a sodium channel antagonist or a pharmaceutically acceptable salt thereof;
- a selective N-methyl D-aspartate (NMDA) receptor antagonist or a pharmaceutically acceptable salt thereof;
- a dopamine agonist or a pharmaceutically acceptable salt thereof;
- a potassium channel opener or a pharmaceutically acceptable salt thereof;
- a calcium channel antagonist or a pharmaceutically acceptable salt thereof;
- an AMPA/kainate receptor antagonist or a pharmaceutically acceptable salt thereof;
- a GABA-A receptor agonist or a pharmaceutically acceptable salt thereof;
- an acetylcholine esterase inhibitor or a pharmaceutically acceptable salt thereof;
- a metalloprotease inhibitor or a pharmaceutically acceptable salt thereof; or
- TPA or a pharmaceutically acceptable salt thereof.

2. The pharmaceutical composition according to claim 1 wherein the neurodegenerative disease is selected from the group consisting of stroke, hypovolemic shock, traumatic shock, reperfusion injury, multiple sclerosis, AIDS, associated dementia, neuron toxicity, Alzheimer's disease, head trauma, adult respiratory disease (ARDS), acute spiral cord injury, Huntington's disease and Parkinson's Disease.

3. Use of an N-NOS inhibitor or a pharmaceutically acceptable salt thereof in combination with at least one of:
- L-Dopa or a pharmaceutically acceptable salt thereof;
- a sodium channel antagonist or a pharmaceutically acceptable salt thereof;
- a selective N-methyl D-aspartate (NMDA) receptor antagonist or a pharmaceutically acceptable salt thereof;
- a dopamine agonist or a pharmaceutically acceptable salt thereof;
- an AMPA/kainate receptor antagonist or a pharmaceutically acceptable salt thereof;
- a potassium channel opener or a pharmaceutically acceptable salt thereof;
- a calcium channel antagonist or a pharmaceutically acceptable salt thereof;
- a GABA-A receptor agonist or a pharmaceutically acceptable salt thereof;
- an acetylcholine esterase inhibitor or a pharmaceutically acceptable salt thereof;
- a matrix metalloprotease inhibitor or a pharmaceutically acceptable salt thereof or;
- TPA or a pharmaceutically acceptable salt thereof, in the manufacture of a medicament for the treatment of neurodegenerative diseases in a mammal.

4. Use according to Claim 3, wherein the neurodegenerative disease is selected from the group consisting of stroke, hypovolemic shock, traumatic shock, reperfusion injury, multiple sclerosis, AIDS, associated dementia, neuron toxicity, Alzheimer's disease, head trauma, adult respiratory disease (ARDS), acute spiral cord injury, Huntington's disease and Parkinson's Disease.

5. Use of a pharmaceutical composition according to Claim 1 or Claim 2 in the manufacture of a medicament for the treatment of neurodegenerative diseases in mammals.
